# EUROPEAN PATENT APPLICATION

(11) **EP 3 872 488 A1**
(43) Date of publication of application: **01.09.2021**
(21) Application number: 19876872.3
(22) Date of filing: 26.09.2019
(51) Int. Cl.: G01N 33/48, G01N 30/88, G01N 33/49, G01N 37/00

(54) **BLOOD PRETREATMENT FOR MICROFLUIDIC BLOOD CELL SORTING**

(30) Priority: 25.10.2018 JP 2018200712
(71) Applicant: TL Genomics Inc., Koganei-shi, Tokyo 184-0012 (JP)
(72) Inventor: KUBO, Tomohiro, Koganei-shi, Tokyo 184-0012 (JP); KANEIWA, Tomoyuki, Koganei-shi, Tokyo 184-0012 (JP); YAMANAKA, Hiroaki, Koganei-shi, Tokyo 184-0012 (JP); SEKI, Ryohei, Koganei-shi, Tokyo 184-0012 (JP)
(74) Representative: Pfenning, Meinig & Partner mbB
(86) International application number: PCT/JP2019/038044
(87) International publication number: WO 2020/085000

(57) **Abstract**

Blood containing cells is brought into contact with a porous surface of a porous material before classification of the cells in the blood by flowing the blood through a microchannel (S80). In an example, the porous material is added to the blood containing the cells and mixed together, thereby bringing the blood containing the cells into contact with the porous surface. In an example, the porous material has particles with the porous surface including polysaccharides. The porous material is added to the blood containing the cells while being suspended in a liquid. In an example, the particles have a predetermined particle size distribution. A median particle size d50V in the volume-based cumulative distribution is 25 to 280 µm.

## Description

### Technical Field

The present invention relates to pretreatment of blood, particularly to pretreatment of blood for classifying blood cells using a microchannel. The present invention also relates to a method for evaluating the pretreatment of blood.

### Background Art

Patent Literature 1 describes classification of blood cells using a microchannel.

### Citation List

### Patent Literature

Patent Literature 1: International Patent Publication No. WO 2018/123220

### Summary of Invention

### Technical Problem

When blood flows through a microchannel, clogging may occur in the microchannel. It is an object of the present invention to provide a method suitable for eliminating such clogging.

### Solution to Problem

<1> A method for pretreating blood, comprising: bringing the blood containing cells into contact with a porous surface of a porous material before flowing the blood through a microchannel to classify the cells in the blood.
<2> The pretreatment method according to <1>, wherein the blood containing the cells is brought into contact with the porous surface by adding the porous material to the blood containing the cells, and mixing them.
<3> The pretreatment method according to <2>, wherein the porous material has particles with the porous surface comprising polysaccharides, and is added as a suspension in a liquid to the blood containing the cells.
<4> The pretreatment method according to <3>, wherein the particles have a particle size distribution and a median particle size d50V in the volume-based cumulative distribution of 25 to 280 µm.
<5> The pretreatment method according to <4>, wherein the porous material is capable of fractionating DNA when the porous material is used for gel filtration chromatography, and the porous material has an exclusion limit for the DNA of 45 base pairs or more.
<6> The pretreatment method according to <4>, wherein the porous material is capable of fractionating a protein when the porous material is used for gel filtration chromatography, and at least any one of the conditions that the porous material has a lower limit of a fractionation range for the protein of 1 × 10⁴ Da or more and that the porous material has an upper limit of the fractionation range for the protein of 4 × 10⁶ Da or more is satisfied.
<7> The pretreatment method according to <4>, wherein small particles having a particle size smaller than or equal to a cutoff diameter are previously removed from the particles, and the cutoff diameter is within a range of 25 to 100 µm.
<8> The pretreatment method according to <3>, wherein the blood to be brought into contact with the surface of the porous material is whole blood that is not diluted with another liquid, and the whole blood is diluted with another liquid after the contact with the surface of the porous material; or the blood to be brought into contact with the surface of the porous material is whole blood previously diluted with another liquid.
<9> A method for classifying cells in blood, comprising: pretreating the blood containing the cells according to the pretreatment method according to <3>; and thereafter flowing the blood pretreated through the microchannel to hydraulically classify the cells in the blood.
<10> The classification method according to <9>, wherein in the pretreatment, the particles have a particle size distribution and a median particle size d50V in the volume-based cumulative distribution of 25 to 280 µm, and small particles having a particle size smaller than or equal to a cutoff diameter are previously removed from the particles by sieving, and in the classification, a flat entry channel that makes the blood flow planar is provided upstream of a point where the hydraulic classification is performed in the microchannel, and the cutoff diameter is larger than a length in a short direction of a cross section of the entry channel.
<11> The classification method according to <10>, wherein a pillar dense area is provided in the entry channel so as to across the blood flow, and each pillar in the pillar dense area stands along the short direction.
<12> The classification method according to <11>, wherein the classification enriches any of fetal nucleated red blood cells (fNRBC), circulating tumor cells (CTCs), and myeloma cells.
<13> A method for evaluating pretreatment of blood, comprising: flowing the blood through a microchannel after the pretreatment of the blood containing cells, wherein a pillar dense area provided in the microchannel so as to across the blood flow; and observing debris spreading in the pillar dense area and a section adjacent to the pillar dense area downstream thereof where pillars are sparse, after a certain time has elapsed from the start of flowing the blood.
<14> The evaluation method according to <13>, wherein the section is surrounded by the most upstream pillar dense area, the pillar dense area located next to the most upstream pillar dense area, and a sidewall of the microchannel, and a ratio of the debris spreading with respect to the section, as the section is seen in planar view, is determined as an area ratio.
<15> The evaluation method according to <14>, wherein the pretreatment is performed by bringing the blood containing the cells into contact with a surface of a test material.
<16> The evaluation method according to <14>, wherein the pretreatment is performed by adding a test material to the blood containing the cells, and mixing.

### Advantageous Effects of Invention

The present invention can provide a method suitable for eliminating clogging in a microchannel.

### Brief Description of the Drawings

[Fig. 1] Fig. 1 is a flow chart including steps of pretreatment.
[Fig. 2] Fig. 2 is a particle size distribution of porous particles.
[Fig. 3] Fig. 3 is a planar view of a microchannel.
[Fig. 4] Fig. 4 is a partial planar view of the microchannel.
[Fig. 5] Fig. 5 is a detailed partial perspective view of the microchannel.
[Fig. 6] Fig. 6 is a perspective view of an entry channel.
[Fig. 7] Fig. 7 is an observation image 1 (upper row) of the microchannel and a sketch thereof (lower row).
[Fig. 8] Fig. 8 is a flow chart of an operation to evaluate the pretreatment.
[Fig. 9] Fig. 9 is an observation image 2 (upper row) of the microchannel and a sketch thereof (lower row).
[Fig. 10] Fig. 10 is a graph 1 of clogging rates.
[Fig. 11] Fig. 11 is a graph 2 of clogging rates.

### Description of Embodiments

### <1. Positioning of method for pretreating blood>

One aspect of the present invention relates to pretreatment of blood. Before describing the details of the pretreatment of blood, positioning of the pretreatment will be described with reference to the drawings. In a figure, a flow chart including step S80 of pretreating blood is shown. Before performing step S80, blood is previously collected from a living body in step S79.

The pretreatment step S80 is carried out before step S81 of classifying cells in blood. Classifying the cells in the blood means to fractionate the cells in the blood depending on their size. Step S81 is particularly performed by flowing the blood through a microchannel. An example of classification in step S81 is hydraulic classification performed within the microchannel. The microchannel has a channel structure on the order of micrometers. Such a microchannel structure is suitable for blood classification (Patent Literature 1). A chip having the microchannel to be used for blood classification may be particularly referred to as a blood cell-separating chip.

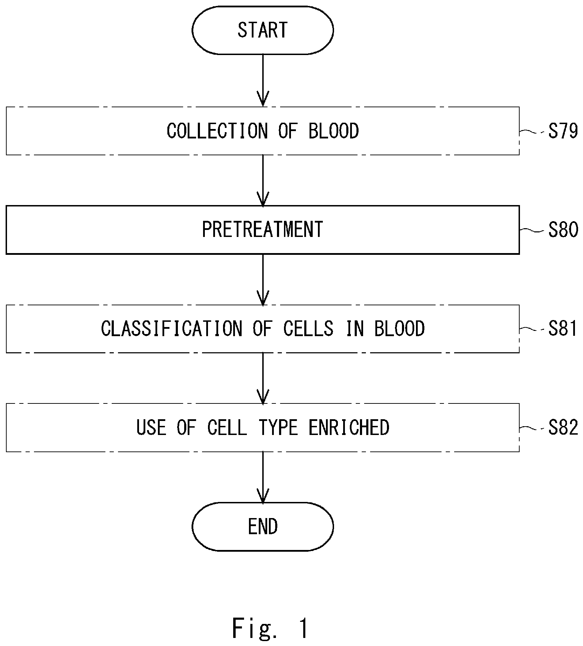

In Fig. 1, a population of cells having a specific particle size distribution is obtained by the classification of step S81. In the cells in the blood, cells of various sizes are mixed. Each cell type has a unique particle size distribution concerning the cell size. Accordingly, a specific cell type is enriched within each population of cells obtained by classification.

In Fig. 1, the cell type enriched in step S82 is used. From the cell type enriched, data that can be used for diagnosis by a doctor can be obtained, for example. The following combinations of the subject to be diagnosed and the cell type to be enriched can be mentioned. These are examples.

The blood targeted in Fig. 1 includes blood cells as the cell type to be enriched. The blood cells may be fetal nucleated red blood cells (fNRBC). The blood to be obtained in step S79 includes fNRBC. fNRBC are contained in maternal blood. Fetuses and pregnant women are subjects to be diagnosed. In step S80, maternal blood is pretreated. In step S81, fNRBC are enriched by classification. In step S82, data useful for diagnosis of fetuses is obtained.

The blood targeted in Fig. 1 includes other cells that circulate in the blood and are not blood cells as the cell type to be enriched. The other cells may be circulating tumor cells (CTCs). The blood to be obtained in step S79 includes CTCs. CTCs may be contained, for example, in the blood of subjects suspected of having cancer, cancer patients, and subjects who have already been treated for cancer. These people are subjects to be diagnosed. In step S80, their blood is pretreated. In step S81, CTCs are enriched by classification. Steps necessary for enriching CTCs are carried out regardless of whether or not CTCs are contained in the blood. In step S82, data useful for diagnosis of cancer is obtained.

The cell type to be enriched that is included in the blood targeted in Fig. 1 may be myeloma cells. The blood to be obtained in step S79 includes myeloma cells. Myeloma cells may be detected as minimal residual disease (MRD), for example, from patients treated for myeloma. These people are subjects to be diagnosed. An example of myeloma is multiple myeloma. In step S80, the blood collected from such a patient is pretreated. In step S81, myeloma cells are enriched by classification. Steps necessary for enriching myeloma cells are carried out regardless of whether or not myeloma cells are contained in the blood. In step S82, data useful for diagnosis of MRD is obtained.

In Fig. 1, step S79, step S81, and step S82 have been described for understanding of the technical meaning of step S80. Accordingly, these steps are not essential in this aspect. Further, another step may be introduced between these steps. The other step may be introduced either before or after step S80.

### <2. Details of method for pretreating blood>

The pretreatment step S80 shown in Fig. 1 will be described further in detail. In step S80, the blood is brought into contact with a porous surface of a porous material. The blood includes blood cells and other cells circulating in blood. The blood includes cells and blood plasma. In order to bring the blood into contact with the porous surface, the porous material is added into the blood. The porous material may be added into the blood collected in a container. The blood may be put into a container in which the porous material has been previously collected. Further, the porous material and the blood are mixed together. These are preferably mixed well so that the porous material is spread throughout the blood.

The porous material may be one that sinks in the blood. The porous material may have particles. The particles may be spherical. The particles may be beads. As used herein, beads refer to a group of particles formed by a technique of forming each particle into a spherical shape. The particles may be suspended in the blood. When the porous material has particles, the porous material may be previously suspended in a liquid other than the blood. The liquid other than the blood may be a buffer or a preservative solution. Serum may be added to the liquid other than the blood. FBS may be used as serum. The blood and the porous material may be brought into contact with each other by adding a suspension of particles of the porous material into the blood.

The blood to be brought into contact with the porous surface may be whole blood that is not diluted with another liquid. The whole blood means blood that is not separated for each blood component and contains all components such as blood cells and blood plasma.

After collecting whole blood in step S79 shown in Fig. 1, the whole blood may be stored for a certain period until the pretreatment in step S80. The storage period may be 1 to 72 hours. The storage temperature may be at room temperature of 1 to 30°C (Japanese Pharmacopoeia). The storage temperature is preferably 4 to 25°C, particularly preferably 4°C. The storage temperature may have a fluctuation in the range of ±2°C. The fluctuation range is preferably ±1°C. When the whole blood is transported outside the blood-collecting facility after the blood collection in step S79 and then pretreated in step S80, the whole blood is preferably transported while being maintained at 4°C. Alternatively, the whole blood may be immediately pretreated in step S80 without interposing the storage step after the whole blood is collected in step S79.

In the pretreatment step S80 shown in Fig. 1, incubation for a predetermined time at a predetermined temperature is preferably performed after the porous material and the blood are mixed together. The time can be, for example, 1 to 60 minutes. The time may be 30 minutes ± 5 minutes. The incubation temperature may be at room temperature of 1 to 30°C (Japanese Pharmacopoeia). The incubation temperature may be 4 to 25°C. The incubation temperature is not particularly limited as long as it does not significantly impair the conditions of the cells or substances in the whole blood. For example, after storing the whole blood in an environment at 4°C, the whole blood that has already been cooled may be incubated in an environment at 25°C.

When the porous material has particles, the amount of the porous material to be added is 10 to 50 µL per 1 mL of undiluted blood. The amount of the porous material to be added may be adjusted depending on the dilution ratio of diluted blood. In this case, the amount of the porous material to be added may be reduced as the dilution ratio increases. For example, when 1 mL of blood is diluted 5-fold, 10 µL of the porous material may be added with respect to 5 mL of the diluted blood. When 1 mL of blood is diluted 2.5-fold, 20 µL of the porous material may be added with respect to 2.5 mL of the diluted blood.

In the aforementioned description, the porous material is supposed to have swollen with water when specifying the volume of the porous material. When the porous material is beads, the porous material may be handled in the form of a bead solution. The bead solution is obtained by mixing the porous material into phosphate buffer normal saline (PBS). Here, the beads of the porous material are weighed out, supposing that gaps between the beads are included in the volume of the beads for convenience. As an example, a bead solution 50% of which is occupied by the beads of the porous material and the remaining 50% of which is occupied by PBS may be used.

In the aforementioned description, the porous material may be previously washed. The washing may be performed using PBS. The washing may be performed using a liquid for diluting blood or distilled water. The washing may be performed twice or 3 times or more.

In Fig. 1, the pretreatment step S80 may be performed simultaneously with the blood collection step S79, for example. For example, the porous material is previously disposed within a blood collection tube. Thereby, the whole blood collected and the porous material are brought into contact with each other within the blood collection tube simultaneously with the blood collection. The blood may be brought into better contact with the porous material by shaking the blood collection tube.

In Fig. 1, the blood that is brought into contact with the porous surface in the pretreatment step S80 may be also whole blood diluted with another liquid. For example, blood collected from a living body is diluted with another liquid after the blood collection step S79 and before the blood is brought into contact with the porous material in step S80. In this description, the blood also includes diluted whole blood. The dilution ratio may be greater than 1-fold and 10-fold or less. That is, another liquid with a volume larger than 0 and 9 or less may be added with respect to whole blood with a volume of 1. The dilution ratio may be any of 1.5-, 2.0-, 2.5-, 3.0-, 3.5-, 4.0-, 4.5-, and 5.0-fold. The dilution ratio may be any of 6-, 7-, 8-, and 9-fold.

In an example, 50 to 100 µL of a bead solution (beads 50 vol%) is added with respect to 1 mL of whole blood, and the mixture is rotated and mixed under an environment at 25°C for 30 minutes. During the rotation and mixing, the beads of the porous material and blood cells are incubated. This is diluted with PBS and subjected to classification of blood cells. In another example, after the whole blood is diluted with PBS, the porous material may be added thereto. In this case, the classification is performed without incubation. Alternatively, the porous material may be previously added into a large tube, and diluted blood may be further added thereto. Further, the whole blood may be diluted and brought into contact with the porous material simultaneously by adding the porous material to a diluting solution previously prepared and adding whole blood thereto.

The porous material may react with components contained in blood plasma. For example, the porous material may adsorb components contained in blood plasma. The components to be adsorbed may be components that directly cause clogging of the microchannel. The components to be adsorbed may be components that indirectly promote clogging of the microchannel.

Multiple micropores are formed on the surface of the porous material. The porous material may be bonded to another non-porous material. For example, non-porous particles coated with a porous material may form porous particles. The center of each particle may be non-porous. The center of each particle may be ferromagnetic.

The material of the porous material may be polysaccharides. The micropores of the porous material may be formed by polysaccharides. The polysaccharide may be crosslinked. The polysaccharides may be any of agarose, dextran, and allyl dextran. The polysaccharides may be modified. The modification may be DEAE (Diethylethanolamine) modification.

The particulate porous material may be a material that can be used for gel filtration chromatography. Gel filtration chromatography is size-exclusion chromatography in which the mobile phase is an aqueous solution. At this time, a material that can fractionate DNA may be employed. The exclusion limit of the porous material for DNA is preferably 45 base pairs or more. The exclusion limit of the porous material for DNA may be 165 base pairs or more or 165 base pairs or less. The exclusion limit of the porous material for DNA may be 1078 base pairs or more or 1078 base pairs or less.

The particulate porous material may be a material that can fractionate a protein. The lower limit of the fractionation range of the porous material with respect to the protein is preferably 1 × 10⁴ Da or more. The upper limit of the fractionation range of the porous material with respect to the protein is preferably 4 × 10⁶ Da or more. The particulate porous material preferably satisfies at least any one of the aforementioned conditions.

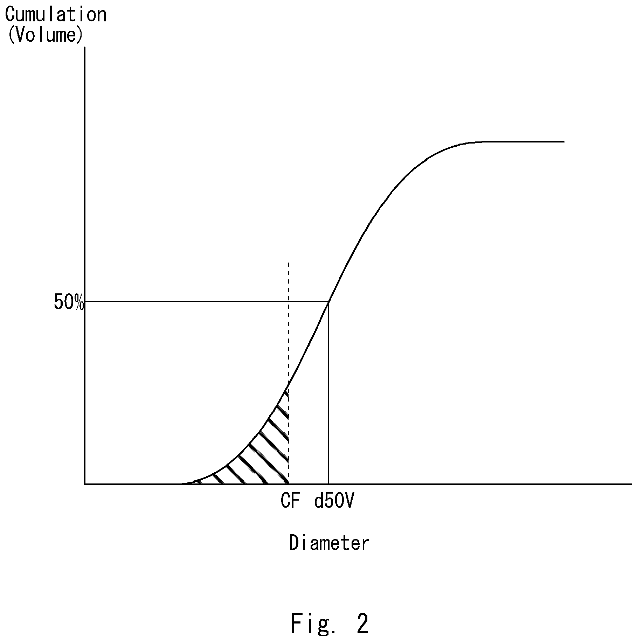

Fig. 2 shows the particle size distribution of the porous particles as a volume-based cumulative distribution. The particles of the porous material have a particle size distribution. The median particle size d50V in the cumulative volume distribution of the porous material is a median particle size in the volume-based cumulative distribution. When the particles are made of polysaccharides, the particle size in the state of the particles swelling in a buffer is used as a reference. A measurement example of the particle size is an effective size determined by laser diffraction or light-scattering or a sphere volume equivalent diameter determined by the Coulter method.

In Fig. 2, the median particle size d50V is preferably less than 500 µm. The median particle size d50V is preferably 25 to 280 µm, more preferably 25 to 165 µm, further preferably 45 to 165 µm. The median particle size d50V falling within such a range enables the surface area of the porous material to be suitable for the contact with the blood.

In Fig. 2, small particles are preferably cut off from the particles of the porous material, as required. The cutoff means to remove small particles from the particles of the porous material. In one aspect, small particles having a particle size of a cutoff diameter CF or less are previously removed. The range of the cutoff diameter CF is 25 to 100 µm. The cutoff diameter CF may be 40 to 70 µm. The removal of small particles is preferably performed by sieving using a mesh. For example, small particles may be removed from a population of the particles of the porous material using a cell strainer. The removal of small particles can suppress clogging in the microchannel due to small particles mixed in the blood. Depending on the type of the microchannel, small particles that have penetrated into the microchannel flow out of the microchannel without incidents. For such a channel, there is no need to consider clogging due to small particles. However, even in such a microchannel, clogging (debris described later) may occur due to some chemical components in the blood. Accordingly, use of particles that have not been cut off is effective for suppressing clogging.

When small particles are removed from the original particles, the particle size distribution of the original particles changes. That is, the cutoff has an effect of size selection. After the size selection, the median particle size also changes. For convenience, the median particle size d50V in this description is based on the particle size distribution before small particles are removed from the original particles by cutoff.

### <3. Method for classifying cells in blood>

Fig. 1 is referred to again. One aspect of the present invention is a method for classifying cells in blood which combines step S80 and step S81. First, blood containing cells is treated based on the aforementioned pretreatment method (step S80). Next, the cells in the blood are hydraulically classified by flowing the pretreated blood through a microchannel (step S81).

In Fig. 1, the blood may be previously diluted and then classified in step S81. The dilution may be performed before or after the pretreatment step S80. The dilution ratio with respect to whole blood in the classification may be larger than 1-fold and 10-fold or less. That is, another liquid with a volume larger than 0 and 9 or less may be added with respect to whole blood with a volume of 1.
The dilution ratio may be any of 1.5-, 2.0-, 2.5-, 3.0-, 3.5-, 4.0-, 4.5-, and 5.0-fold. The dilution may be performed both before and after the pretreatment.
For example, the whole blood may be diluted 2 to 3-fold before the pretreatment and further diluted 5-fold after the completion of the pretreatment with respect to the whole blood.

After step S80 of pretreating the blood in Fig. 1, the pretreated blood may be stored until the classification in step S81. The range of the storage temperature after the pretreatment may be 4°C ±2°C. The classification may be performed in step S81 immediately after the pretreatment without interposing the storage step.

### <4. Classification device>

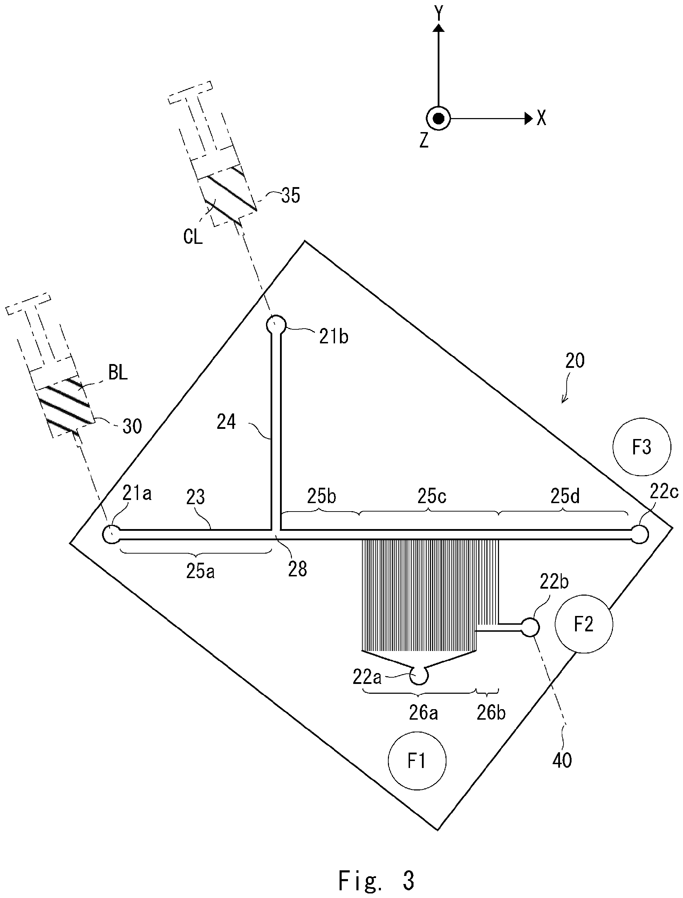

Hereinafter, an example of a device for hydraulic classification is shown. In Fig. 3, a microchannel 20 in planar view is shown. The microchannel 20 is a channel chip for separating floating cells including blood cells. The axes X, Y, and Z in the figure are each shown for convenience of description, in order to understand the functions of the microchannel 20. These axes do not specifically limit the shape of the microchannel 20.

In Fig. 3, the microchannel 20 has a main channel 23. One end of the main channel 23 serves as an inlet 21a. The other end of the main channel 23 serves as an outlet 22c. The microchannel 20 further has a sub-channel 24. An end of the sub-channel 24 serves as an inlet 21b. The other end of the sub-channel 24 is connected to the main channel 23 at a junction 28.

In Fig. 3, the main channel 23 has channel parts 25a to 25d provided sequentially from the inlet 21a toward the outlet 22c. The channel parts 25a to 25d are connected into one piece from the inlet 21a to the outlet 22c. The junction 28 is interposed between the channel part 25a and the channel part 25b.

In Fig. 3, the microchannel 20 has branch channels 26a and 26b. Both the branch channels 26a and 26b are channels branched from the main channel 23. The branch channel 26a and the branch channel 26b are branched from the main channel 23 in this order sequentially from the upstream side. One end of each of the branch channels 26a and 26b is connected to the main channel 23 in the channel part 25c. In the channel part 25c, the branch channels 26a and 26b are disposed on the side opposite to the sub-channel 24. An outlet 22a and an outlet 22b are present at the other ends of the branch channels 26a and 26b.

In Fig. 3, the branch channels 26a and 26b each have a plurality of small channels branched from the main channel 23. The small channels are each aligned along the direction from the upstream to the downstream of the main channel 23. The small channels reach the outlets 22a and 22b, respectively. The small channels merge before the outlets 22a and 22b respectively. The channel part 25d is present downstream of the channel part 25c. The channel part 25d reaches the outlet 22c.

In Fig. 3, the inlet 21a is connected to a syringe 30 containing pretreated blood BL. The blood BL is sent from the syringe 30 to the inlet 21a at a predetermined flow rate. The blood BL enters the channel part 25a through the inlet 21a. The blood BL may be pretreated within the syringe 30. After the blood BL is pretreated within the syringe 30, the blood BL is sent from the syringe 30 to the microchannel 20.

In Fig. 3, the microchannel 20 has the sub-channel 24. The sub-channel 24 is connected to a syringe 35. A clarified liquid CL is put into the syringe 35. The clarified liquid CL is a liquid free from floating cells. The clarified liquid CL is a liquid that does not damage blood cells and other cells. The clarified liquid CL is a buffer. The clarified liquid CL also may be PBS. The clarified liquid CL enters the sub-channel 24 through the inlet 21b by applying pressure to the syringe 35. The clarified liquid CL flows through the sub-channel 24. The clarified liquid CL flows into the channel part 25b.

A fraction of the cell suspension is discharged through each outlet. A fraction F3 at the outlet 22c, a fraction F2 at the outlet 22b, and a fraction F1 at the outlet 22a are respectively obtained. The fraction F1 and the fraction F2 each contain cells classified in the channel part 25c. The fraction F3 contains blood plasma that has passed through the channel part 25c.

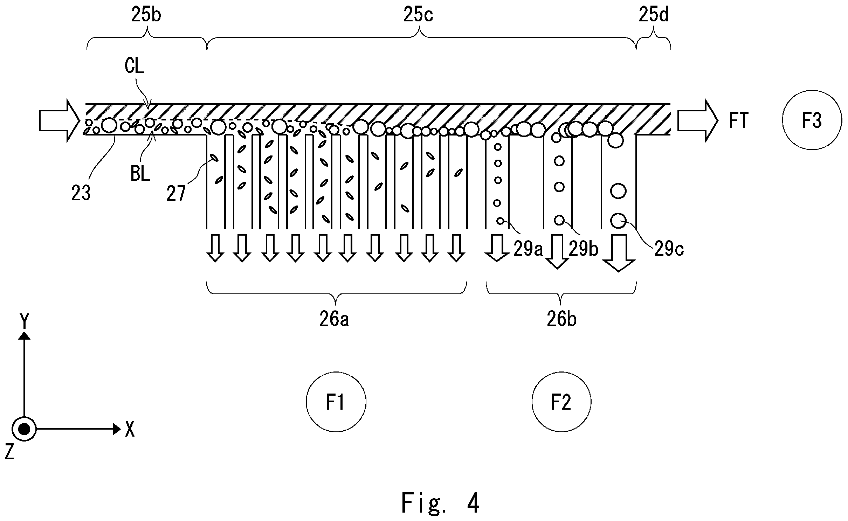

Fig. 4 shows the microchannel 20 in planar view. The figure schematically shows the process of fractionating floating cells using the microchannel 20. For the sake of simplicity, the branch channel 26a shows 10 small channels, and the branch channel 26b shows 3 small channels.

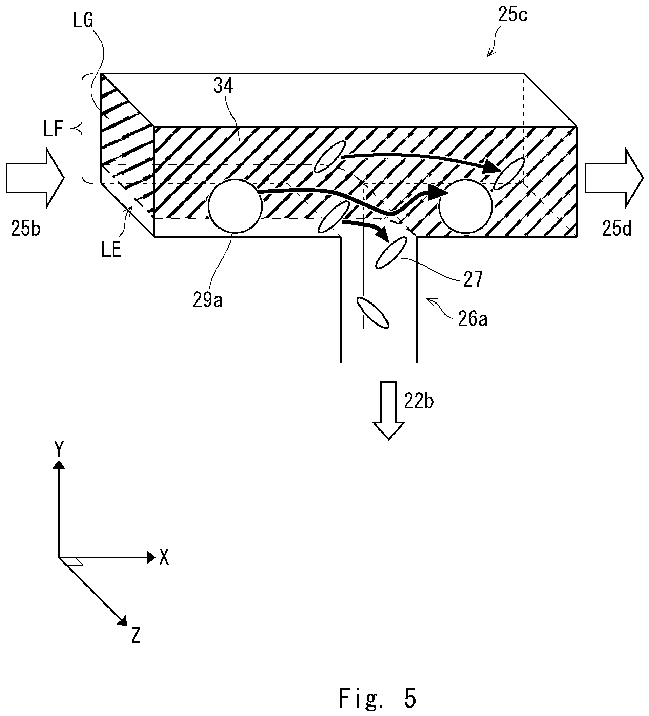

Fig. 4 and Fig. 5 are cited from Japanese Unexamined Patent Application Publication No. 2007-175684 and partially changed. The mechanism of classification is described particularly in detail in Japanese Unexamined Patent Application Publication No. 2007-175684.

As shown in Fig. 4, the blood BL continuously flows from further upstream of the channel part 25b. The blood BL contains a large amount of cells. The flow of the clarified liquid CL is continuously brought into contact with the flow of the blood BL from a lateral side thereof. Thereby, cells flowing through the main channel 23 are continuously pushed away from the lateral side of the main channel 23. As a result, the flow of the blood BL is continuously pushed toward the opposite side of the flow of the clarified liquid CL. In the channel parts 25b to 25c, floating cells are continuously pushed toward the side of the branch channels 26a and 26b, and the floating cells continuously flow into these branch channels.

As shown in Fig. 4, non-nucleated red blood cells 27 continuously flow into the branch channel 26a. In the channel part 25b, the non-nucleated red blood cells 27 in the blood BL are hydraulically classified. The classification is continuously performed in the flow of the blood BL on the side that is not in contact with the flow of the clarified liquid CL.

As shown in Fig. 4, the branch channel 26a functions as a channel to remove the non-nucleated red blood cells 27. The inscribed circle diameter of each small channel of the branch channel 26a is 12 to 19 µm. The inscribed circle diameter may be any of 13, 14, 15, 16, 17, and 18 µm.

As shown in Fig. 4, nucleated cells 29a to 29c continuously flow into the branch channel 26b. In the channel part 25c downstream of the channel part 25b, the nucleated cells 29a to 29c in the blood BL are hydraulically classified. The classification is continuously performed in the flow of the blood BL on the side that is not in contact with the flow of the clarified liquid CL. In particular, a cell suspension of nucleated cells is continuously obtained from the branch channel 26b.

As shown in Fig. 4, the branch channel 26b functions as a channel for collecting the nucleated cells 29a to 29c. The inscribed circle diameter of each small channel of the branch channel 26b is 20 to 30 µm. The inscribed circle diameter may be any of 21, 22, 23, 24, 25, 26, 27, 28, and 29 µm. The diameter of nucleated cells including nucleated red blood cells is considered to be 11 to 13 µm.

The inscribed circle diameter of each small channel of the branch channels 26a and 26b shown in Fig. 4 is the diameter of an inscribed circle in a cross section orthogonal to the small channel. The shape of the cross section of the small channel may be square, other polygonal, or circular. The same applies to other branch channels. The floating cells and blood plasma that have not been taken into the branch channels 26a and 26b continuously pass through the channel part 25d as a flow-through FT. Thereafter, they reach the outlet 22c in Fig. 3. For example, the flow-through FT includes aggregated blood cells and the like.

Fig. 5 shows a detail of the microchannel focusing on the channel part 25c. In the hydraulic classification according to this example, the value of the inscribed circle diameter of the small channel is not equal to the maximum value of the diameter of the floating cells to be classified. Accordingly, the hydraulic classification according to this example is different from simple filtration. The hydraulic classification according to this example will be described below.

Fig. 5 shows the channel part 25c. Fig. 5 further shows a small channel of the branch channel 26a. For the sake of simplicity, only one small channel constitutes the branch channel 26a. In the description of Fig. 5, the small channel constituting the branch channel 26a is simply referred to as the branch channel 26a.

As shown in Fig. 5, a liquid flow LF is continuously introduced into the channel part 25c. The liquid flow LF includes the blood BL and the clarified liquid CL described above. These liquids are partially mixed at the interface of the liquid flow LF. The nucleated cells 29a as large cells and the non-nucleated red blood cells 27 as small cells are contained in the liquid flow LF. In the figure, the nucleated cells 29a are depicted as a representative of other nucleated cells.

In Fig. 5, part of the liquid flow LF that is introduced into the branch channel 26a is referred to as a liquid flow LE. Part of the liquid flow LF that is not introduced into the branch channel 26a and flows downstream is referred to as a liquid flow LG. In the liquid flow, only the liquid flow LG is hatched. Here, the flow rate of the liquid flow LE is proportional to the cross section of the liquid flow LE. The liquid flow LE flows along the inner wall on the branch channel 26a side of the channel part 25c. The flow rate of the liquid flow LE is also proportional to the flow rate of the liquid flow LE in the branch channel 26a.

In Fig. 5, the non-nucleated red blood cells 27 flowing within the liquid flow LE are introduced into the branch channel 26a. Meanwhile, more than half of the volume of the nucleated cells 29a belong to the liquid flow LG side. The nucleated cells 29a only partially contact the liquid flow LE. Therefore, the nucleated cells 29a are not introduced into the branch channel 26a. At this time, the diameter of the nucleated cells 29a may be smaller than the inscribed circle diameter of the branch channel 26a. If the flow rate of the liquid flow LE increases, the cross section of the liquid flow LE increases. In this case, it is also conceivable that the nucleated cells 29a are taken into the liquid flow LE and guided to the branch channel 26a.

In Fig. 5, the nucleated cells 29a are carried further downstream by the liquid flow LG. Thus, a fluid that does not contain floating cells of a certain size or more can be collected from the branch channel 26a. As a result, the non-nucleated red blood cells 27 are classified herein. Further, the nucleated cells 29a and other nucleated cells are classified downstream.

### <5. Entry channel>

Fig. 3 is referred to again. The channel part 25a in the microchannel 20 is located upstream of the channel part 25c where hydraulic classification is performed. The channel part 25a is located upstream of the junction 28 where the flow of the clarified liquid CL further merges. The channel part 25a is connected to the inlet 21a. Hereinafter, the channel part 25a may be referred to as an entry channel.

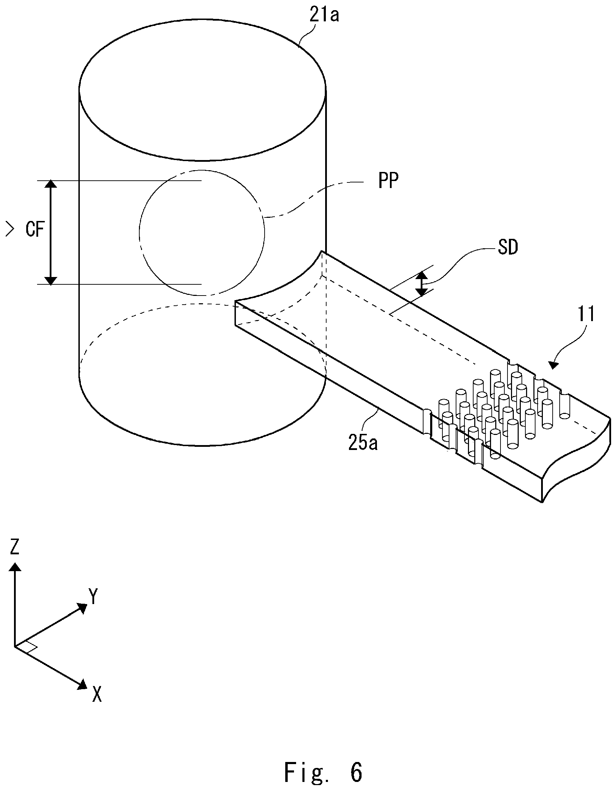

Fig. 6 shows the channel part 25a that corresponds to the entry channel. The blood penetrates through the inlet 21a into the channel part 25a. The channel part 25a is flat. The flow of the blood in the channel part 25a is made planar. The length in the short direction of a cross section YZ of the channel part 25a is expressed as a dimension SD.

According to Fig. 6, the dimension SD corresponds to the height of the channel part 25a. The dimension SD is preferably 25 ± 5 µm. Selecting a suitable value for the dimension SD facilitates the flow of cells in the blood cells. Further, the cells in the blood cells can be spread in the Y direction in the channel part 25a. Further, the dimension SD is preferably maintained throughout the other channel parts. With such a configuration, the flow of the clarified liquid from the +Y direction facilitates pushing the flow of blood in the +X direction toward the -Y direction (see Fig. 4).

Fig. 2 is referred to again. As described above, the porous particles used for the pretreatment have a particle size distribution. In one aspect, the median particle size d50V is less than 500 µm. The median particle size d50V is preferably 25 to 280 µm, more preferably 25 to 165 µm, further preferably 45 to 165 µm.

In Fig. 2, small particles having a cutoff diameter CF or less are preferably previously removed from the particles by sieving in the pretreatment as described above. The range of the cutoff diameter CF is 25 to 70 µm, preferably 25 to 40 µm.

Fig. 6 shows porous particles PP. The particles PP have a particle size larger than the cutoff diameter CF. In one preferable aspect, the cutoff diameter CF is larger than the dimension SD. In other words, almost all the particles PP have a particle size larger than the dimension SD. Here, even if the particles PP having a particle size smaller than the dimension SD remain, these particles are considered to be accidentally incorporated, for example, due to incomplete sieving.

In Fig. 6, the particles PP can be prevented from entering the channel part 25a by setting the size of the particles PP as described above. In one preferable aspect, a pillar dense area 11 is provided in the course of the channel part 25a. Each pillar in the pillar dense area 11 connects the upper surface and the lower surface of the channel part 25a. The particles PP can be prevented from blocking the pillar dense area 11 by setting the size of the particles PP as described above. The configuration and functions of the pillar dense area 11 are described below.

### <6. Pillar dense area>

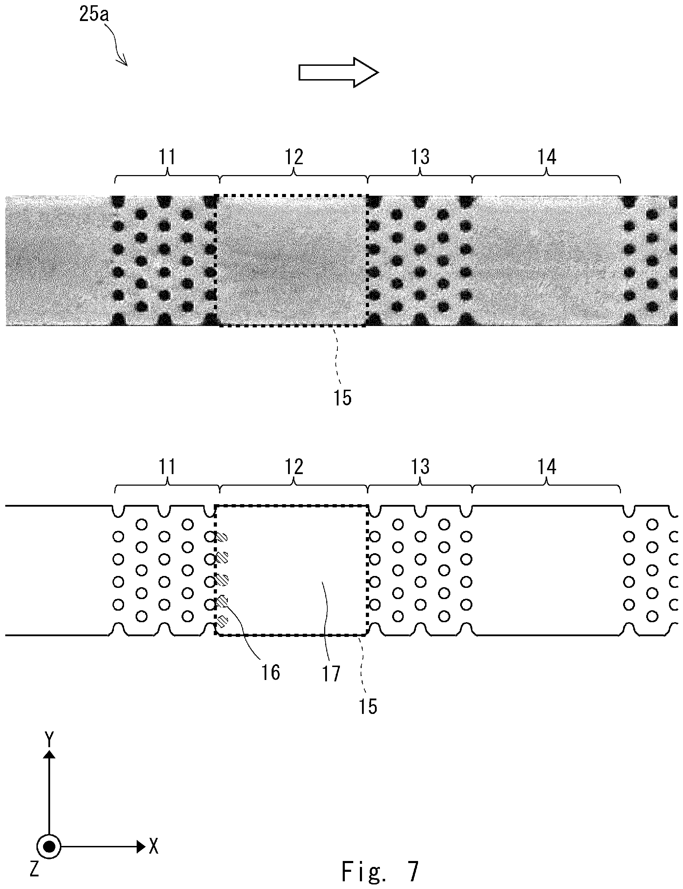

Fig. 7 shows an observation image of the channel part 25a and a sketch thereof. The channel part 25a includes the pillar dense area 11. The pillar dense area 11 is provided so as to across the blood flow in the channel part 25a. The blood flows from upstream on the left side to downstream on the right side in the figure.

In Fig. 7, the channel part 25a has a section 12. The section 12 is adjacent to the pillar dense area 11 downstream of the pillar dense area 11. In the section 12, pillars are sparse. The channel part 25a further includes a pillar dense area 13. The pillar dense area 13 is a pillar dense area located next to the pillar dense area 11 in the downstream direction as viewed from the pillar dense area 11. The configuration of the pillar dense area 13 may be the same as that of the pillar dense area 11.

In Fig. 7, the section 12 is surrounded by the pillar dense area 11, the pillar dense area 13, and a sidewall of the channel part 25a, that is, a sidewall of the microchannel. The channel part 25a further has a section 14. The section 14 is adjacent to the pillar dense area 13 downstream of the pillar dense area 13. The channel part 25a further includes a pillar dense area downstream of the section 14. The reference numerals 15 to 17 will be described in Examples.

In Fig. 7, the pillar dense area 11 does not provide a great resistance to the blood flow. Further, the pillar dense area 11 does not significantly reduce the blood flow rate. The pillar dense area 11 plays a role of a filter. The pillar dense area 11 prevents impurities in the blood such as insoluble components larger than blood cells from entering the channel parts downstream of the pillar dense area 11. Further, if there are aggregated blood cells, the pillar dense area 11 plays a role of breaking apart the blood cells one by one.

### <7. Example of pretreatment>

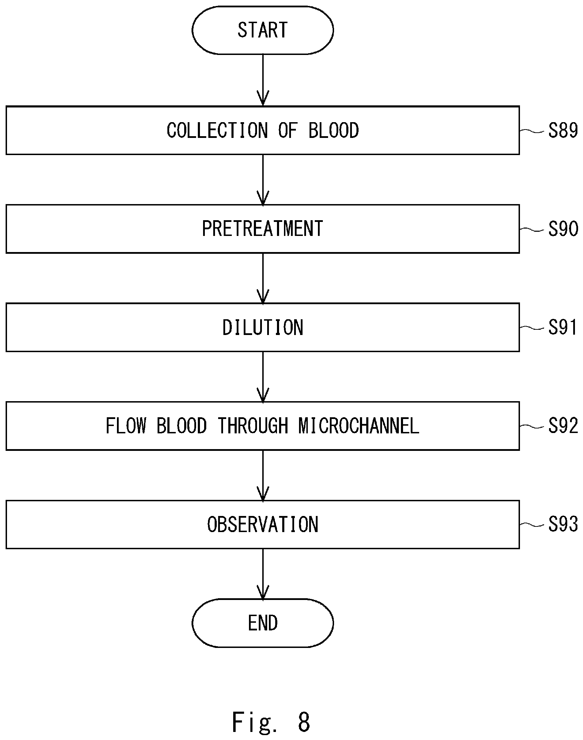

In this example, an example of a method for pretreating blood and its effects will be described based on Fig. 7 to Fig. 11. Fig. 7 will be described below. Fig. 8 is a flow chart of an operation to evaluate the pretreatment. In step S89, blood is collected from a human body. The blood is stored at 4°C for 1 to 48 hours before the pretreatment step S90.

In step S90 in Fig. 8, the blood is pretreated with polysaccharide beads of the porous material. For this, the beads are previously washed. First, the beads diluted 2-fold with PBS are added to a 1.5-mL tube. After mixing PBS with the beads, the supernatant is discarded by centrifugation at 500 × g for 2 minutes at RT (at room temperature: 25°C). Further, washing by adding PBS is repeated, to perform the washing operation 3 times in total. After the washing, the mixture is diluted with PBS to the original volume at the time of the 2-fold dilution. The mixture of PBS and the beads is added to the blood as a bead solution.

In Fig. 8, the blood is pretreated in step S90 by adding the beads to the blood. In Examples, porous polysaccharide beads B01, B02, and B03 are each added to the blood and mixed. The amount of the beads to be added is 50 µL per 1 mL of the diluted blood. The mixing is performed by rotation at 25°C for 30 minutes. After a lapse of 30 minutes, the rotation is stopped, and the mixture is further left standing for 30 minutes at RT. The list of the beads is as follows.

**[Table 1]**

| Beads | Product name | DNA exclusion limit | Protein fractionation range |
|---|---|---|---|
| B01 | Sepharose CL-6B | 45 - 165 bp | 1 × 10⁴ - 4 × 10⁶ Da |
| B02 | Sephacryl-500-HR | 1078 bp | 4 × 10⁴ - 2 × 10⁷ Da |
| B03 | Sephadex G-75 | 20 - 25 bp, estimated | 3 × 10³ - 8 × 10⁴ Da |

The beads were all obtained from Merck (Sigma-Aldrich). Sepharose, Sephacryl, and Sephadex shown in the column of the product name are all trademarks.

The DNA exclusion limit (Fractionation Range [Mr] DNA Exclusion Limit) is described as follows. That is, solute particles larger than the maximum pore diameter of the beads are excluded from the micropores. When DNA is selected as the solute particles, there is a limit of exclusion based on the number of bases. Mr represents a relative molecular mass.

The protein fractionation range is a fractionation range for the molecular size (Da) of globular proteins (Fractionation range [Mr], Globular proteins).

The gel matrix of the beads B01, Sepharose CL-6B, is a sphere made of 6% crosslinked agarose. The particle size is 45 to 165 µm.

The gel matrix of the beads B02, Sephacryl 500-HR (product number: S500HR, available from Sigma-Aldrich) is made of a crosslinked copolymer of allyldextran and N,N'-methylenebisacrylamide. The median particle size in the cumulative volume distribution (Particle size, dsov) is 50 µm or less. The particle size is 25 to 75 µm. The fractionation range based on dextran (Fractionation [Mp] Dextrans) is 4 × 10⁴ to 2 × 10⁷. Mp represents a peak molecular weight. The exclusion limit with respect to dextran is 100 × 10⁶.

The gel matrix of the beads B03, Sephadex G-75, is made of crosslinked dextran. The particle size in a wet state is 90 to 280 µm. The particle size in a dry state is 40 to 120 µm. The fractionation range based on dextran (Fractionation [Mp] Dextrans) is 1 × 10³ -5 × 10⁴. The exclusion limit with respect to dextran is larger than 7 × 10⁴. The DNA exclusion limit of Sephadex G-50 is 20 bp. The DNA exclusion limit of Sephadex G-100 is 25 bp. Sephadex G-75 is considered to have an intermediate DNA exclusion limit therebetween. Therefore, the DNA exclusion limit of Sephadex G-75 is estimated to be in the range of 20 or more and 25 or less.

Next, the blood is diluted with PBS in step S91 shown in Fig. 8. The dilution ratio is 2 to 3-fold.

Next, the blood flows through the blood cell-separating chip (microchannel) in step S92 shown in Fig. 8. No treatment was performed to remove the beads from the blood.

Next, the chip is observed in step S93 shown in Fig. 8. The observation is performed 30 to 90 minutes after the blood has started to flow in step S92. The observation is performed while the blood is flowing through the blood cell-separating chip.

Fig. 7 is referred to again. The section 12 is observed. The chip is disposed under a USB camera (HOZAN TOOL IND. CO., LTD). The chip is observed in planar view. The chip is captured with a HOZAN USB cam software to obtain the image data shown in the upper row of Fig. 7. The image data is read into ImageJ software and analyzed on the same software.

An area 15 that corresponds to the section 12 is cut out from the image data in Fig. 7. The total number of pixels in the area 15 is regarded as the total area of the section 12. Debris parts 16 in the area 15 are distinguished from a flow part 17 by visual inspection. The debris parts 16 are occupied by debris accumulated from the pillar dense area 11 as starting points. The debris spreading to the pillar dense area 11 causes clogging in the pillar dense area 11. The flow of blood cells is pushed away by the debris part 16. In contrast, blood cells smoothly flow in the flow part 17.

In Fig. 7, lines are visually drawn between the debris parts 16 and the flow part 17. The number of pixels within the debris parts 16 defined by the lines is determined. The sum total of the number of pixels of all the debris parts 16 within the section 12 is determined as a debris area. The debris area with respect to the total area of the section 12 is determined as a percentage. Such a value is used as a clogging rate.

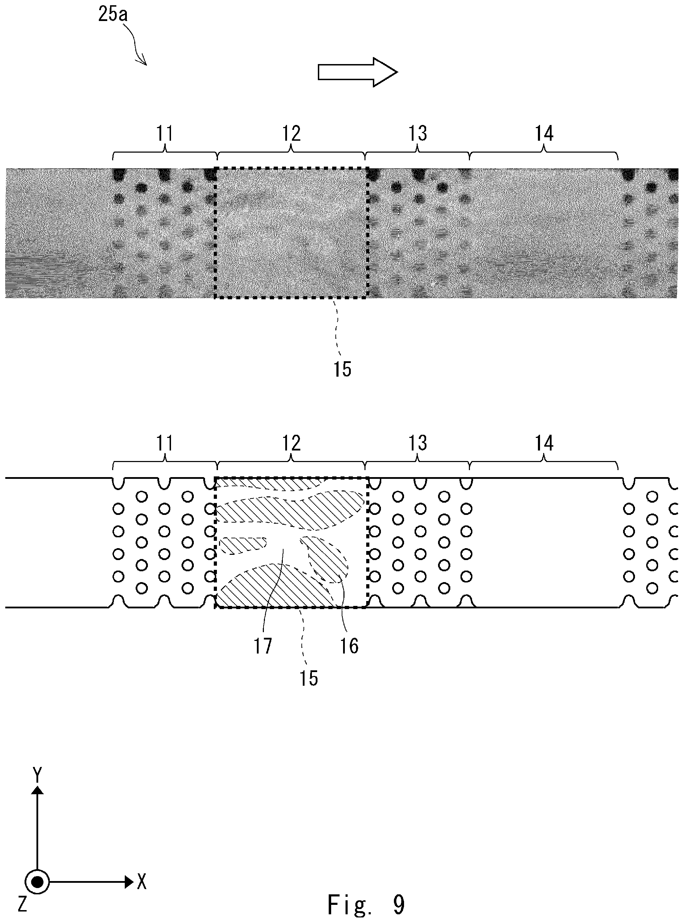

Fig. 9 shows an observation image of the channel part 25a in Comparative Example C01 and a sketch thereof. In Comparative Example C01, blood is prepared in the same manner as in Examples above except that no beads are added. The blood flows through the blood cell-separating chip in the same manner as above. As the observation was also performed in the same manner, the debris parts 16 spreading to most of the area 15 (70 % or more). Further, the debris was subdivided.

In Comparative Example C01 shown in Fig. 9, the method for calculating the clogging rate is changed. The sum total of the number of pixels of the portion excluding the debris parts 16 from the area 15, that is, the flow part 17 is obtained. This is specified as an area of the flow part 17. The area of the flow part 17 is subtracted from the total area of the area 15, and thus the area of the debris parts 16 is estimated. The estimated area is used as a debris area.

The area 15 shown in Fig. 9 coincides with the entire range of the section 12. The area 15 may be a partial range of the section 12.

In Fig. 9, the debris penetrates toward the inside of the pillar dense area 11 over the boundary between the section 12 and the pillar dense area 11. Alternatively, it is also inferred that the debris generated inside the pillar dense area 11 continues to the section 12.

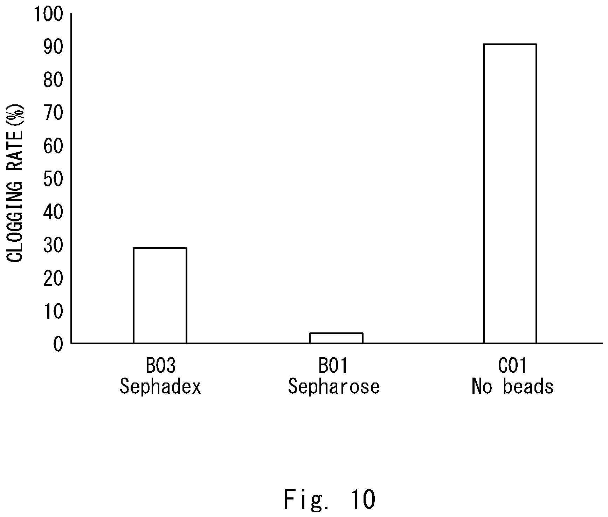

Fig. 10 is graph 1 showing clogging rates. The blood pretreated with the beads B01 and the beads B03 have a clogging rate lower than that of the blood of Comparative Example C01. Accordingly, it was found that polysaccharide beads having a porous surface give an effect to prevent clogging.

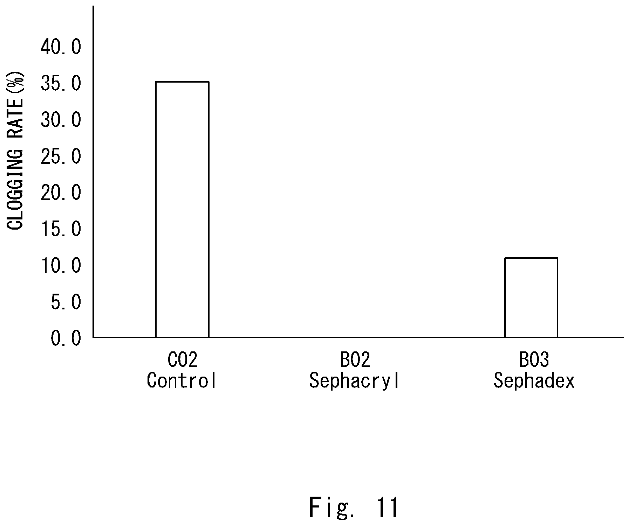

Fig. 11 is graph 2 showing clogging rates. The blood pretreated with the beads B02 and the beads B03 have a clogging rate lower than that of the blood of Comparative Example C02 (Control). It could also be confirmed from the results of Fig. 11 that polysaccharide beads having a porous surface give an effect to prevent clogging. In Control of Comparative Example C02, the blood is merely mixed by rotation without adding a mixture of beads and PBS to the blood.

As shown in Fig. 10 and Fig. 11, the beads B01 and the beads B02 have a stronger effect to prevent clogging than the beads B03. As shown in Table 1, the beads B01 and the beads B02 have a higher DNA exclusion limit than the beads B03. Further, in the beads B01 and the beads B02, the protein fractionation range is biased toward the larger protein side than in the beads B03.

As Comparative Example C03, pretreatment was performed in the same manner as above by mixing non-porous agarose powder with the blood. As Comparative Example C04, pretreatment was performed by bringing the blood into contact with the surface of a slant-type jelly of an agarose gel formed by dissolving agarose powder. In both Comparative Examples, no remarkable effects to prevent clogging could be found as compared with the case where no pretreatment was performed. It has been found that the porosity of the material used for pretreatment is more important to obtain an effect to prevent clogging than the chemical properties of polysaccharides.

As another example, the dilution in step S91 is performed earlier before the pretreatment in step S90 in Fig. 8. That is, the order of step S90 and step S91 is reversed. The beads B01 (Sepharose CL-6B) are used. Also in this case, an effect to prevent clogging could be confirmed.

<8. Example of combining pretreatment and classification>

### (1) Cutoff and pretreatment

In this example, small particles are cut off from the porous particles for pretreatment. In this example, the beads B01, Sepharose CL-6B, are used. 1 mL of the beads are put into a nylon cell strainer (FALCON, trademark). Under stirring with a stirrer, the beads are filtered overnight. The filtration time may be several hours to 24 hours. The filtration is performed in distilled water.
The mesh size of the cell strainer is 40 µm, 70 µm, and 100 µm. The filtration is performed for the cell strainer of each mesh size. Here, a usage example of the 70-µm mesh cell strainer will be described in detail. The mesh size corresponds to the cutoff diameter. Large particles filtered by the cutoff are used for pretreatment. As the mesh size decreases, the yield of large particles increases. The beads are washed twice with PBS.

Each beads are suspended in a clarified liquid in the same volume as that of the beads. 20 µL of the suspension of the beads is added to 2 mL of the clarified liquid, and these are mixed well. The clarified liquid is PBS previously supplemented with 1% FBS. The mixed solution of the beads and the clarified liquid is further added to a clarified liquid. The inside of the blood cell-separating chip is previously immersed in the buffer by previously flowing a part of the clarified liquid through the blood cell-separating chip. The immersing the blood cell-separating chip is performed for 40 minutes.

### (2) Evaluation of cutoff diameter

The blood cell-separating chip after the classification is observed with a microscope. In the case of pretreatment with beads having cutoff diameters of 70 µm and 100 µm, debris was observed inside the entry channel (the channel part 25a) or in the vicinity of the inlet 21a as shown in Fig. 6. The channel was slightly closed by the debris. In contrast, in the case of pretreatment with beads having a cutoff diameter of 40 µm, no debris could be found. Further, the classification of the sample blood was continued for 2 hours and 40 minutes, but no closure of the channel was observed. It was found that it is desirable to adjust the cutoff diameter to 70 µm or less, in order to obtain an effect to suppress closing of the channel by debris. The cutoff diameter may be 60 µm or 50 µm.

In any case of the cutoff diameters of 40 µm, 70 µm, and 100 µm, the entire amount of the sample blood could be processed by the blood cell-separating chip. There was no excess sample blood that could not be fractionated due to the blood cell-separating chip being closed by debris. In the following tests, the cutoff diameter of 70 µm will be employed.

### (3) Cell spike

In this example, K562 cells are spiked into whole blood to form a model for CTCs. The whole blood is collected from a healthy human and stored at 4°C. A suspension of K562 cells is added to the whole blood one day after collecting the blood, to obtain sample blood. The amount to be added is 865.4 cells with respect to 2 mL of the blood. The K562 cells are lymphoid buoyant cultured cells derived from a patient with chronic myeloid leukemia.

The number of K562 cells to be added is previously estimated as follows. First, the K562 cells are Hoechst-stained. After the staining, the cells are observed with a microscope to count the number of cells. As described above, the number of K562 cells per volume of the suspension of K562 cells can be checked.

The sample blood is diluted 5-fold with a clarified liquid (1% FBS-PBS) mixed with the beads described above. The sample blood is pretreated by being brought into contact with the beads. Blood cells are classified by immediately flowing the sample diluted blood through the blood cell-separating chip. As the results of the classification are described with reference to Fig. 3 and Fig. 4, a large number of white blood cells containing lymphocytes are obtained in the fraction F2. A large number of non-nucleated mature red blood cells are obtained in the fraction F1. Most (> 99.9%) of the cells flowing into the blood cell-separating chip flow out to the fraction F1. A large number of blood cells are not obtained in F3. Since the K562 cells are lymphoid, they are expected to be contained in the fraction F2.

### (4) Quantitative evaluation of classification

Table 2 shows the results of quantifying the classification. The upper row represents the case where the beads were not brought into contact with the sample blood (Beads less). The lower row represents the case where the beads cut off at 70 µm were brought into contact with the sample blood (Cut off). The elapsed time after the classification was started (Time course, minutes) is divided into three time intervals of 0 to 15 minutes, 15 to 45 minutes, and 45 to 75 minutes.

**[Table 2]**

| | | Whole blood | | | | | K562 cells | | | |
|---|---|---|---|---|---|---|---|---|---|---|
| | Time course (min) | Input cell number (x 10⁹) | Input volume (mL) | Output volume (F1,F2,F3) (mL) | Output cell number (F1,F2,F3) (x 10⁹) | Rate of collection | Input cell conc. (Cells/mL) | Input cell number | Output cell number (F2,F3) | Rate of collection |
| Beads less | 0 - 15 | 3.18 | 0.075 | 0.065 | 3.56 | 111.9% | 443.0 | 33.23 | 34 | 102.3% |
| | 15 - 45 | 6.36 | 0.150 | 0.146 | 8.67 | 136.3% | 443.0 | 66.45 | 91 | 136.9% |
| | 45 - 75 | - | - | - | - | - | - | - | - | - |
| Cut off | 0 - 15 | 3.18 | 0.075 | 0.065 | 3.39 | 106.6% | 432.7 | 32.45 | 26 | 80.1% |
| | 15 - 45 | 6.36 | 0.150 | 0.153 | 7.92 | 124.5% | 432.7 | 64.91 | 57 | 87.8% |
| | 45 - 75 | 6.36 | 0.150 | 0.155 | 8.66 | 136.2% | 432.7 | 64.91 | 54 | 83.2% |

The total number of cells in the sample blood containing blood cells in the whole blood is shown on the left side in Table 2. The number of cells flowing into the blood cell-separating chip (Input cell number) is obtained by measuring the number of blood cells per unit volume (mL) of the diluted blood. The measurement is performed by processing 10 µL of the whole blood diluted with PBS at a predetermined dilution ratio using a TC20 Automated Cell Counter (BIO RAD). Further, calculation is performed by multiplying the dilution ratio and the volume of the whole blood with the measured value obtained.

The number of cells in the sample blood flowing into the blood cell-separating chip (Input cell number) is shown on the left side in Table 2. Further, the volume of the sample blood flowing into the blood cell-separating chip (Input volume) is shown there, converted into whole blood.

Further, the total number of cells flowing out to the fractions F1, F2, and F3 (Output cell number) (× 10⁹) is shown in Table 2. The ratio of the total number of outflow cells (Output cell number) with respect to the number of inflow cells (Input cell number) is shown as a rate of collection (%). In Table 2, there are some samples with a rate of collection (%) exceeding 100%. This is inferred that the small volume of the whole blood fractionated caused errors in dilution at the time of measurement of the number of cells or measurement of the number of cells.

Cells in whole blood are classified without any contact with the beads. In this case, clogging occurs within the blood cell-separating chip. Accordingly, it is difficult to continue the classification of cells over 45 minutes. Meanwhile, almost all the cells in the whole blood brought into contact with the beads that have been cut off are collected.

### (5) Evaluation of rate of collection of nucleated cells

The total number of cells in the sample blood containing blood cells in the K562 cells spiked is shown on the right side in Table 2. The number of K562 cells flowing out to the fraction F2 and the fraction F3 is actually measured. In the actual measurement of the number of cells, it is necessary to distinguish the K562 cells from other nucleated cells derived from whole blood. This is performed by staining the K562 cells with Hoechst33342 (available from Sigma-Aldrich) before previously spiking, observing fractionated cells collected after fractionation with an all-in-one fluorescence microscope BZ-X710, available from KEYENCE CORPORATION, and distinguishing cells whose nuclei are fluorescently labeled as the K562 cells and cells without such a label as the other nucleated cells derived from whole blood.

The value obtained (Output cell number) is used for calculating the rate of collection. Here, the rate of collection is the number of K562 cells actually collected within the fraction F2 and the fraction F3 with respect to the number of K562 cells flowing into the blood cell-separating chip (Input cell number) expressed as a percentage.

The number of K562 cells flowing into the blood cell-separating chip (Input cell number) is described as follows. In the test without beads (Beads less), 443 K562 cells per 1 mL of whole blood used for preparing the sample blood were mixed with the sample blood. This value is shown as a concentration of inflow cells (Input cell conc.) in Table 2. The volume of the sample blood fractionated (Input cell volume) 0 to 15 minutes after the start of the classification is 0.075 mL. However, this value has been converted into the volume of whole blood in consideration of dilution. The number of inflow cells (Input cell number) in this time interval is 443 × 0.075 = 33.23. Assuming that all the K562 cells are collected in any one of the fraction F2 and the fraction F3 in this time interval, 33.23 K562 cells are collected. In this time interval (Time course, 0 to 15 minute) without beads (Beads less), the number of K562 cells flowing out to the fraction F2 and the fraction F3 (Output cell number) is 34. This value is a measured value of the number of K562 cells collected. The rate of collection of the K562 cells is 34/33.23 × 100 = 102.3%.

In Table 2, the volume of the sample blood fractionated (Input cell volume) is 0.150 mL 15 to 45 minutes after the start of the classification. However, this value has been converted into the volume of whole blood in consideration of dilution. The number of inflow cells (Input cell number) in this time interval is 443 × 0.150 = 66.45. The number of K562 cells flowing out to the fraction F2 and the fraction F3 (Output cell number, measured value) is 91. The rate of collection of the K562 cells is 91/66.45 × 100 = 136.9%.

The rate of collection of the K562 cells when the cutoff diameter is 70 µm is further shown on the lower row of Table 2. The number of K562 cells measured found within fractions composed of the fraction F2 and the fraction F3 (Output cell number) was 26, 57, and 54 in each time interval. In the pretreatment with the beads with a cutoff diameter of 70 µm, the rate of collection of the K562 cells was 80 % or more at any time interval, and therefore the rate of collection is determined to be sufficiently high.

This example is performed as a model experiment of enriching CTCs. The results above indicate that the pretreatment is useful for enriching CTCs. Specifically, it is indicated that long-term classification can be performed by preventing the clogging of the chip. A large amount of the sample blood can be processed by performing long-term classification by the method of this example. This means that the amount of CTCs obtained per process is large. Further, these are considered to be also effective for enriching cells contained in blood in only a small amount, for example, fetal erythroblasts in maternal blood, like CTCs.

### <9. Method for evaluating pretreatment>

Another aspect of the present invention is a method for evaluating the pretreatment of blood. Fig. 8 is referred to again, and a description will be given with reference to the figure. Blood containing cells is collected in step S89. The blood containing cells is pretreated in step S90.

In the pretreatment in step S90, the blood containing cells is brought into contact with the surface of the test material. The test material may be in powder form. The test material may be a structure formed on the surface of a container. Alternatively, the test material is added to the blood containing cells and mixed therewith. The test material may be dissolved in the blood.

As shown in Fig. 8, the blood is diluted in step S91. The dilution ratio is preferably at least 2-fold. The dilution ratio is, for example, 2 to 3-fold. The dilution is performed, for example, with PBS. The dilution may be performed before step S90. That is, the order of step S90 and step S91 may be reversed. The dilution may be performed before step S90, and further dilution may be performed in step S91.

In step S92, the blood is allowed to flow through the microchannel. As shown in Fig. 7, the pillar dense area 11 is provided in the microchannel so as to across the flow of blood. The debris part 16 occurring in the pillar dense area 11 and in the section 12 adjacent to the pillar dense area 11 downstream thereof is observed. In the section 12, there are no pillars, or pillars are sparse.

The debris part 16 occurring in the section 12 shown in Fig. 7 is observed after a certain time has elapsed from the start of flowing the blood. The time is, for example, 30 to 90 minutes. The ratio of the area occupied by the debris part 16 in the area 15 is determined as an area ratio. The determination method may be according to Examples above.

It should be noted that the present invention is not limited to the aforementioned embodiments and can be appropriately changed without departing from the gist.

This application claims priority to Japanese Patent Application No. 2018-200712, filed on October 25, 2018, the disclosure of which is incorporated herein by reference in its entirety.

### Reference Signs List

11: Pillar dense area
12: Section
13: Pillar dense area
14: Section
15: Area
16: Debris part
17: Flow part
20: Microchannel
21a and 21b: Inlets
22a to 22c: Outlets
23: Main channel
24: Sub-channel
25a to 25d: Channel parts
26a and 26b: Branch channels
27: Non-nucleated red blood cells
28: Junction
29a to 29c: Nucleated cells
30: Syringe
35: Syringe
B01 to B03: Beads
BL: Blood
C01 to C04: Comparative Examples
CF: Cutoff diameter
CL: Clarified liquid
d50V: Median particle size
LE: Liquid flow
LF: Liquid flow
LG: Liquid flow
PP: Particles
S79 to S82: Steps
S89 to S93: Steps
SD: Dimension

## Claims

1. A method for pretreating blood, comprising
bringing the blood containing cells into contact with a porous surface of a porous material before flowing the blood through a microchannel to classify the cells in the blood.

2. The pretreatment method according to claim 1, wherein
the blood containing the cells is brought into contact with the porous surface by adding the porous material to the blood containing the cells and mixing them.

3. The pretreatment method according to claim 2, wherein
the porous material has particles with the porous surface comprising polysaccharides, and is added as a suspension in a liquid to the blood containing the cells.

4. The pretreatment method according to claim 3, wherein
the particles have a particle size distribution and a median particle size d50V in the volume-based cumulative distribution of 25 to 280 µm.

5. The pretreatment method according to claim 4, wherein
the porous material is capable of fractionating DNA when the porous material is used for gel filtration chromatography, and the porous material has an exclusion limit for DNA of 45 base pairs or more.

6. The pretreatment method according to claim 4, wherein
the porous material is capable of fractionating a protein when the porous material is used for gel filtration chromatography, and at least any one of the conditions that the porous material has a lower limit of a fractionation range for the protein of 1 × 10⁴ Da or more and that the porous material has an upper limit of the fractionation range for the protein of 4 × 10⁶ Da or more is satisfied.

7. The pretreatment method according to claim 4, wherein
small particles having a particle size smaller than or equal to a cutoff diameter are previously removed from the particles, and
the cutoff diameter is within a range of 25 to 100 µm.

8. The pretreatment method according to claim 3, wherein:
the blood to be brought into contact with the surface of the porous material is whole blood that is not diluted with another liquid, and the whole blood is diluted with another liquid after the contact with the surface of the porous material; or
the blood to be brought into contact with the surface of the porous material is whole blood previously diluted with another liquid.

9. A method for classifying cells in blood, comprising:
pretreating the blood containing the cells according to the pretreatment method according to claim 3; and thereafter
flowing the blood pretreated through the microchannel to hydraulically classify the cells in the blood.

10. The classification method according to claim 9, wherein
in the pretreatment,
the particles have a particle size distribution and a median particle size d50V in the volume-based cumulative distribution of 25 to 280 µm, and
small particles having a particle size smaller than or equal to a cutoff diameter are previously removed from the particles by sieving, and
in the classification,
a flat entry channel that makes the blood flow planar is provided upstream of a point where the hydraulic classification is performed in the microchannel, and
the cutoff diameter is larger than a length in a short direction of a cross section of the entry channel.

11. The classification method according to claim 10, wherein
a pillar dense area is provided in the entry channel so as to across the blood flow, and
each pillar in the pillar dense area stands along the short direction.

12. The classification method according to claim 11, wherein
the classification enriches any of fetal nucleated red blood cells (fNRBC), circulating tumor cells (CTCs), and myeloma cells.

13. A method for evaluating pretreatment of blood, comprising:
flowing the blood containing cells through a microchannel after the pretreatment of the blood, wherein a pillar dense area provided in the microchannel so as to across the blood flow, and
observing debris spreading in the pillar dense area and a section adjacent to the pillar dense area downstream thereof where pillars are sparse after a certain time has elapsed from the start of flowing the blood.

14. The evaluation method according to claim 13, wherein
the section is surrounded by the most upstream pillar dense area, the pillar dense area located next to the most upstream pillar dense area, and a sidewall of the microchannel, and
a ratio of the debris spreading with respect to the section, as the section is seen in planar view, is determined as an area ratio.

15. The evaluation method according to claim 14, wherein
the pretreatment is performed by bringing the blood containing the cells into contact with a surface of a test material.

16. The evaluation method according to claim 14, wherein
the pretreatment is performed by adding a test material to the blood containing the cells and mixing.
